# FASCICULE DE BREVET EUROPEEN

(11) **EP 1 242 083 B1**
(45) Date de publication et mention de la délivrance du brevet: **14.03.2007**
(21) Numéro de dépôt: 00988915.5
(22) Date de dépôt: 14.12.2000
(51) Int. Cl.: A61K 31/4468, A61P 1/00, C07D 211/58, C07D 401/04

(54) **PHENOXYPROPANOLAMINES, PROCEDE POUR LEUR PREPARATION ET COMPOSITIONS PHARMACEUTIQUES LES CONTENANT**
PHENOXYPROPANOLAMIN-DERIVATE, IHRE HERSTELLUNG UND THERAPEUTISCHE VERWENDUNG
PHENOXYPROPANOLAMINES, METHOD FOR PRODUCING THEM AND PHARMACEUTICAL COMPOSITIONS CONTAINING THEM

(30) Priorité: 17.12.1999 FR 9915931; 17.12.1999 FR 9915932
(43) Date de publication de la demande: 25.09.2002
(73) Titulaire: Sanofi-Aventis, 75013 Paris (FR)
(72) Inventeur: CECCHI, Roberto, I-26900 Lodi (IT)
(74) Mandataire: Varady, Peter
(86) Numéro de dépôt international: PCT/FR2000/003535
(87) Numéro de publication internationale: WO 2001/043744

(56) Documents cités:
- EP-A- 0 095 454
- WO-A-99/65895
- DE-A- 3 524 955
- US-A- 5 627 196
- M HORI ET AL: "A soluble polymer approach to the fishing out principle: synthesis and purification of beta-amino alcohols" JOURNAL OF ORGANIC CHEMISTRY,US,AMERICAN CHEMICAL SOCIETY. EASTON, vol. 63, no. 3, 6 février 1998 (1998-02-06), pages 889-894-894, XP002110346 ISSN: 0022-3263 cité dans la demande
- DATABASE CHEMABS [en ligne] CHEMICAL ABSTRACTS SERVICE, COLUMBUS, OHIO, US; ISHIKAWA, MINORU ET AL: "Preparation of aminopiperidine moiety-containing heterocyclic compounds as integrin.alpha.v.beta.3 antagonists" retrieved from STN Database accession no. 131:299455 XP002138383 & WO 99 52872 A (MEIJI SEIKA KAISHA, LTD., JAPAN) 21 octobre 1999 (1999-10-21)

## Description

La présente invention concerne de nouvelles phénoxypropanolamines, les compositions pharmaceutiques les contenant, un procédé pour leur préparation et des intermédiaires dans ce procédé.

BE 902897 décrit des aryloxypropanolamines portant un groupe 4-pipéridininyl-1-substitué sur l'amine, ces composés ayant une activité bêta-1-bloquante et alpha-bloquante.

J. Org. Chem., 1988, 63:889:894, décrit d'autres aryloxypropanolamines portant un groupe 4-pipéridinyl-1-substitué sur l'amine.

Il a été maintenant trouvé que de nouvelles phénoxypropanolamines portant un 4-pipéridinyl-1-susbstitué sur l'amine possèdent une activité agoniste vis-à-vis des récepteurs bêta-3-adrénergiques.

Le récepteur bêta-3 adrénergique a fait l'objet de nombreuses études visant à synthétiser des composés agonistes vis-à-vis de ce récepteur, ces composés exerçant un effet anti-obésité et anti-diabétique important chez l'homme, comme décrit par exemple par Weyer, C et al., Diabetes Metab., 1999, 25(1) :11-21.

Ainsi, la présente invention concerne, selon un de ses aspects, des phénoxypropanolamines de formule (I) 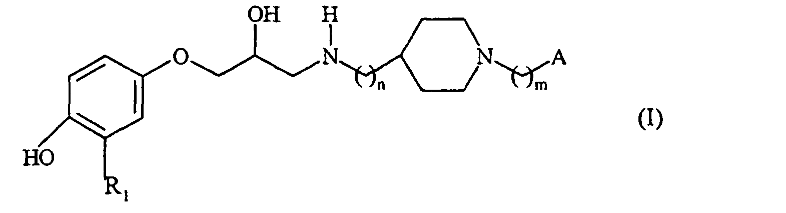 où
- R₁: représente un atome d'hydrogène ou d'halogène, un groupe -S(O)_{Z}-(C₁-C₄)alkyle, -S(O)_{Z}-(C₁-C₄)R₃, -SO₂-NH-(C₁-C₄)alkyle, -NHCO(C₁-C₄)alkyle, -CO(C₁-C₄)alkyle ou -NHSO₂-(C₁-C₄)alkyle ;
- m et n: représentent indépendamment 0, 1 ou 2;
- A: représente un groupe de formule (a) ou (b) : 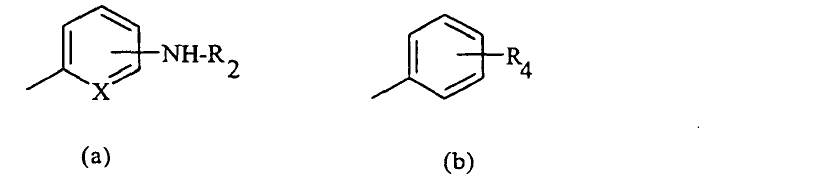
où
- X: est N ou CH ;
- R₂: représente un groupe -SO₂-R₃, -CO-R₃ ou -CO-(C₁-C₄)alkyle ;
- R₃: représente un groupe phényle, éventuellement substitué par un groupe (C₁-C₄)alkyle, (C₁-C₄)alkoxy, un ou deux atomes d'halogène ou un hétérocycle ;
- R₄: représente un atome d'hydrogène ou d'halogène, un groupe (C₁-C₆)alkyle, (C₁-C₄)alcoxy, -COOH, -COO(C₁-C₄)alkyle, -CN, -CONR₅R₆, -NO₂, -NHSO₂(C₁-C₄)alkyle ou - SO₂NR₅ R₆ ;
- z: est 1 ou 2;
- R₅ et R₆: représentent indépendamment un atome d'hydrogène ou un groupe (C₁-C₄)alkyle, phényle ou phényl(C₁-C₄)alkyle ;
et leurs sels ou solvates.

Dans la présente description, les termes "(C₁-C₄)alkyle" et "(C₁-C₆)alkyle" désignent des radicaux monovalents d'un hydrocarbure respectivement en C₁-C₄ et C₁-C₆ à chaîne saturée droite ou ramifiée.

Dans la présente description, le terme "halogène" désigne un atome choisi parmi le chlore, le brome, le iode et le fluor.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphosulfoniques et les acides mandéliques ou mandéliques substitués.

Lorsque les composés de formule (1) possèdent un groupe carboxy libre les sels comprennent aussi les sels avec des bases minérales, de préférence celles avec des métaux alcalins tels que le sodium ou le potassium, ou avec des bases organiques.

Les stéréoisomères optiquement purs, ainsi que les mélanges d'isomères des composés de formule (I), dus aux carbones asymétriques ou au groupe sulfinyle, lorsque z est 1 dans la signification de R₁, dans une proportion quelconque, font partie de la présente invention.

Des composés préférés sont ceux où le groupe (C₁-C₄)alkyle est un groupe méthyle ou éthyle.

D'autres composés préférés encore sont ceux où n et m sont zéro.

Des composés préférés de la présente invention comprennent les composés de formule (I) où A est un groupe (a), X est N et le groupe NHR₂ est dans la position 5 de la pyridine.

D'autres composés préférés de la présente invention comprennent les composés de formule (I) où A est un groupe (b) et le groupe R₄ est dans la position 4 du benzène.

D'autres composés préférés sont ceux où A est un groupe (b) et R₄ est choisi parmi -COOH, -COO(C₁-C₄)alkyle, -CN, -NO₂, -CONR₂R₃, -NHSO₂-(C₁-C₄)alkyle, -SO₂NR₅R₆.

Les composés de formule (I) peuvent être préparés en traitant un composé de formule (II) 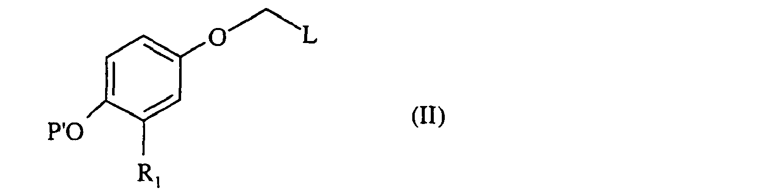 dans laquelle R₁ est tel qu'indiqué ci-dessus, P' est un groupe protecteur et L est un groupe de formule (c) ou (d) 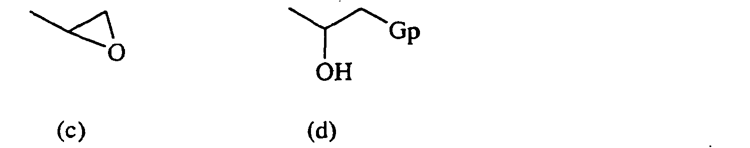 où Gp est un groupe partant tel que le tosylate, le mésylate ou un atome d'halogène, avec une amine de formule (III) 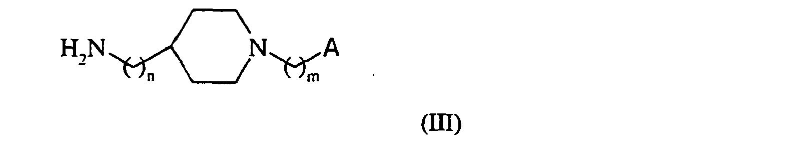 dans laquelle n et m sont tels que définis ci-dessus, en clivant le groupe P' selon les méthodes usuelles et éventuellement en transformant le composé de formule (I) ainsi obtenu en l'un de ses sels.

Plus particulièrement, la réaction entre les composés de formule (II) et (III) est réalisée dans un solvant organique, tel qu'un alcool inférieur comme le méthanol, l'éthanol et l'isopropanol; le diméthylsulfoxyde; un éther linéaire ou cyclique; un amide comme le diméthylformamide ou le diméthylacétamide; en utilisant des quantités au moins équimoléculaires des réactifs, éventuellement en faible excès d'amine.

La température de la réaction est comprise entre la température ambiante et la température de reflux du solvant choisi.

Comme groupes protecteurs P', on peut utiliser les groupes protecteurs usuels pour les groupes hydroxy tels que par exemple le méthoxyéthoxyméthyle (MEM), le benzyle, le benzoyle ou les silyléthers tels que par exemple le *tert-*butyldiméthylsilyléther (TBDMS).

Le clivage de ces groupes protecteurs est effectué selon les méthodes habituelles selon le groupe protecteur choisi et selon la réactivité des autres groupes présents, dans le cas du groupe benzyle, par exemple, par hydrogénation en présence d'un catalyseur tel que le Pd/C dans un solvant convenable ; dans le cas du MEM ou du TBDMS on peut également utiliser un acide tel que l'acide trifluoroacétique; dans le cas du benzoyle on peut faire une réaction de transesterification avec un alcanol en milieu basique.

Les époxydes de formule (II) sont des composés connus en littérature ou bien ils peuvent être préparés par des procédés analogues à ceux décrits dans la littérature. Certains époxydes de formule (II) sont par exemple décrits dans WO 96/04233 et dans US 4,396,629.

Certaines des amines de formule (III) sont des composés nouveaux et constituent un autre aspect de la présente invention.

Ainsi selon un autre de ses aspects, la présente invention concerne des amines de formule (III') 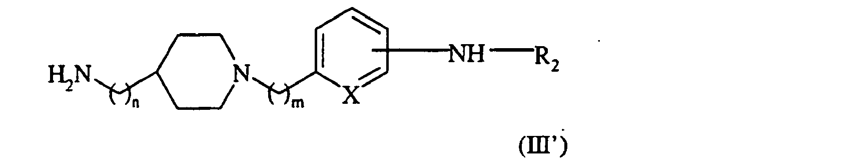 où n, m, X et R₂ sont tels que définis ci-dessus, ainsi que leurs sels ou solvates.
Ces amines peuvent être préparées par réaction des composés de formule (IV) 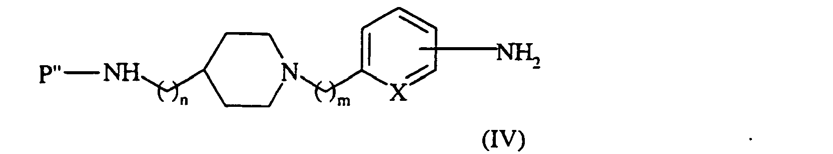 dans laquelle P" est un groupe protecteur tel *tert*-butoxycarbonyle ou le carbobenzyloxy avec un radical CI-R₂ où R₂ est tel que décrit précédemment et Hal est un atome d'halogène, dans un solvant convenable, tel que par exemple la pyridine, le diméthylformamide ou le diméthylsulfoxide et par élimination du groupe P" par hydrogénation ou par traitement en milieu acide tel que l'acide chlorhydrique dans l'éthyle acétate ou dans l'éthanol.

Les amines de départ de formule (IV) peuvent être préparées par réaction des pyridines convenables de formule (V) 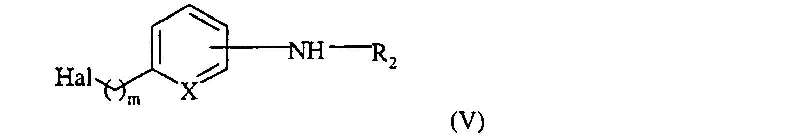 où Hal représente un atome d'halogène et R₂ et m sont tels que définis ci-dessus, avec une pipéridine de formule (VI) ci-dessous 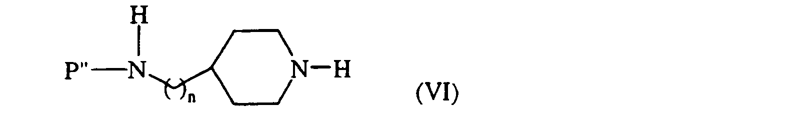 où n est tel que défini ci-dessus et P" représente un groupe protecteur, dans un solvant organique en présence d'une base.

Comme solvant de réaction, on peut bien utiliser par exemple le diméthylformamide, la pyridine, le diméthylsulfoxyde, un éther linéaire ou cyclique ou un solvant chloruré tel que le dichlorométhane.

Comme base on peut utiliser par exemple un hydroxyde alcalin, un carbonate alcalin tel que le carbonate de potassium ou une amine tertiaire telle que la triéthylamine.

La réaction de condensation ci-dessus est complétée en quelques heures, normalement en 2-12 heures.

La température de réaction est comprise entre la température ambiante et la température de reflux du solvant choisi.

Comme groupes protecteurs P", on peut utiliser par exemple les groupes protecteurs indiqués pour les produits de formule (IV).

Le clivage de ces groupes protecteurs est effectué selon les méthodes habituelles décrites le groupe protecteur choisi; dans le cas du *tert*-butoxycarbonyle par exemple, le clivage est normalement effectué par hydrolyse acide.

D'autres intermédiaires nouveaux qui font partie de la présente invention sont les amines de formule (III") 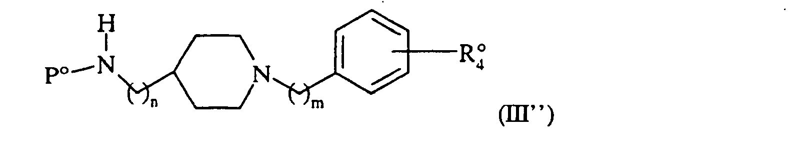 où
où
- P^{o}: est un groupe *tert*-butoxycarbonyle;
- n et m: sont 0, 1 ou 2;
- R^{o} ₄: est un groupe choisi parmi -COO(C₁-C₄)alkyle, -CONR^{o} ₅R^{o} ₆ et -NHSO₂(C₁-C₄)alkyle;
- R^{o} ₅ et R^{o} ₆: représentent indépendamment un atome d'hydrogène ou un groupe (C₁-C₄)alkyle ;
ainsi que leurs sels ou solvates.

Des composés de formule (III") particulièrement préférés sont ceux où n est 0, m est 0 ou 1 et R^{o} ₄ est -COO(C₁-C₄)alkyle.

Les composés de formule (III") peuvent être préparés de façon analogue aux composés (IV) ci-dessus.

Les composés de formule (I) ont montré une affinité très puissante vis-à-vis des récepteurs bêta-3.

L'activité des composés de la présente invention vis-à-vis de l'activité agoniste bêta-3 a été mise en évidence à l'aide d'essais in vitro sur le colon humain selon la méthode décrite dans EP-B-436435 et dans T. Croci et al, Br. J. Pharmacol., 1997, 122: 139P.

Plus particulièrement, on a constaté que les composés de formule (I) sont beaucoup plus actifs sur le côlon isolé que sur l'oreillette et sur la trachée.

Ces propriétés surprenantes des composés de formule (I) permettent d'envisager leur utilisation comme médicaments à action bêta-3.

De plus, les composés de formule (I) sont peu toxiques; notamment, leur toxicité aiguë est compatible avec leur utilisation comme médicaments pour le traitement de maladies dans lesquelles les composés ayant une affinité pour le récepteur bêta-3 trouvent leur application. Les composés de formule (I), ainsi que leurs sels pharmaceutiquement acceptables, peuvent donc être indiqués par exemple dans le traitement des maladies gastro-intestinales telles que le syndrome du colon irritable (IBD), comme modulateurs de la motricité intestinale, comme lipolytiques, agents anti-obésité, anti-diabétiques, psychotropes, anti-glaucomateux, cicatrisants, anti-dépressifs, tocolytiques.

L'utilisation des composés de formule (I) ci-dessus, ainsi que celle de leurs sels et solvates pharmaceutiquement acceptables pour la préparation de médicaments ci-dessus, constitue un aspect ultérieur de la présente invention.

Les composés de formule (I) ci-dessus et leurs sels et solvates pharmaceutiquement acceptables peuvent être utilisés à des doses journalières de 0,01 à 20 mg par kilo de poids corporel du mammifère à traiter, de préférence à des doses journalières de 0,1 à 10 mg/kg. Chez l'être humain, la dose peut varier de préférence de 0,5 mg à 1500 mg par jour, notamment de 2,5 à 500 mg selon l'âge du sujet à traiter, le type de traitement, prophylactique ou curatif, et la gravité de l'affection. Les composés de formule (I) sont généralement administrés en unité de dosage de 0,1 à 500 mg, de préférence de 0,5 à 100 mg de principe actif, une à cinq fois par jour.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ci-dessus ou un de ses sels et solvates pharmaceutiquement acceptables.

Dans les compositions pharmaceutiques de la présente invention pour l'administration orale, sublinguale, sous-cutanée, intramusculaire, intraveineuse, topique, transdermique ou rectale, les ingrédients actifs de formule (I) ci-dessus, leurs sels et solvates pharmaceutiquement acceptables peuvent être administrés sous formes unitaires d'administration, en mélange avec des supports pharmaceutiques classiques, aux animaux et aux êtres humains, pour le traitement des affections susdites. Les formes unitaires d'administration appropriées comprennent les formes par voie orale telles que les comprimés, les gélules, les poudres, les granulés et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration locale, on mélange le principe actif dans un excipient pour la préparation de crèmes ou onguents ou on le dissout dans un véhicule pour l'administration intraoculaire, par exemple sous forme de collyre.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butyléneglycol,

Le principe actif peut être formulé également sous forme de microcapsules, éventuellement avec un ou plusieurs supports ou additifs.

Les composés de formule (I), notamment les composés (I) marquées par un isotope, peuvent aussi être utilisés comme outils de laboratoire dans des essais biochimiques.

Les composés de formule (I) se lient au récepteur bêta-3-adrénergique. On peut donc utiliser ces composés dans un essai ordinaire de liaison ("binding"), dans lequel on emploie un tissu organique où ce récepteur est particulièrement abondant, et on mesure la quantité de composé (I) déplacé par un composé test, pour évaluer l'affinité dudit composé vis-à-vis des sites de liaison de ce récepteur particulier.

Les exemples qui suivent illustrent mieux l'invention.

### PREPARATION 1

### 4-tert-Butoxycarbonylamino-pipéridine.

On mélange à la température ambiante pendant 2 heures 25 g (0,13 mole) de 4-amino-1-benzylpipéridine, 36,2 ml (0,26 mole) de triéthylamine et 31,2 g (0,143 mole) de di-*tert*-butyl-dicarbonate dans 200 ml de diméthylformamide. On verse le mélange dans de l'eau, on extrait à l'acétate d'éthyle, on lave à l'eau et on cristallise le produit ainsi obtenu dans 200 ml d'éther isopropylique. On obtient 33 g de 1-benzyl-4-tert-butoxycarbonylamino-pipéridine qu'on hydrogène dans un mélange de 200 ml d'éthanol et 100 ml de tétrahydrofurane en présence de 3 g de Pd/C à 10%. Après filtration du catalyseur, on isole le composé du titre.

P.f. 157-160°C.

### PREPARATION 2

### 4-tert-Butoxycarbonylamino-1-(4-éthoxycarbonylphenylméthyl)-pipéridine

On chauffe sous agitation pendant 6 heures à 50°C un mélange de 2,01 g (0,010 mole) du produit obtenu dans la Préparation 1 et 2 g (0,010 mole) de 4-chlorométhyl-éthoxycarbonylbenzene dans 40 ml de diméthylformamide. On verse dans de l'eau, on extrait à l'acétate d'éthyle et on lave avec de l'eau. Le produit est filtré et séché. Le brut ainsi obtenu est purifié par flash-chromatographie en éluant par un mélange cyclohexane/acétate d'éthyle = 1 : 1. On obtient le composé du titre. P.f. 74-76°C.

### PREPARATION 3

### 4-Amino-1-(4-éthoxycarbonylphenylméthyl)-pipéridine

Le produit obtenu par la Préparation 2 est chauffé au reflux pendant 5 heures dans une solution contenant 15 ml de acétate d'éthyle et 15 ml d'acide chlorhydrique en acétate d'éthyle (à peu près 3N). Après refroidissement, on filtre, on lave à l'acétone et on sèche le produit sous pression réduite. Le produit du titre est obtenu sous forme de chlorhydrate dihydraté par cristallisation dans une solution d'éthanol.

P.f. 290-293°C.

### PREPARATION 4

### 4-tert-Butoxycarbonylamino-1-(4-éthoxycarbonylphenyl)-pipéridine

On chauffe à 80°C pendant 55 heures 21,6 g (0,10 mole) du produit de la Préparation 1 avec 9,06 g (0,01 mole) de (4-éthoxycarbonyl-1-fluoro)benzène et 14,9 g de K₂CO₃ dans 200 ml de diméthylformamide. On filtre le K₂CO₃. on verse la solution dans l'eau, on extrait à l'acétate d'éthyle et on évapore le solvant. Le brut de réaction est purifié par flash-chromatographie en éluant par un mélange cyclohexane/acétate d'éthyle = 8:2. On obtient le produit du titre qui est cristallisé an acétate d'éthyle.

P.f. 138°-140°C.

### PREPARATION 5

### Chlorhydrate de 4-amino-1-(4-éthoxycarbonylphenyl)-pipéridine.

On dissout 7,94 g (0,023 mole) du produit de la Préparation 4 dans 60 ml d'acétate d'éthyle et on ajoute 80 ml d'une solution 3N d'acide chlorhydrique en acétate d'éthyle. On chauffe à reflux pendant 5 heures on évapore le solvant, on ajoute de l'acétone et on filtre. On obtient le produit du titre qui cristallise en éthanol.

P.f. 240-242°C (chlorhydrate)

### PREPARATION 6

### 4-tert-Butoxycarbonylamino-1-(4-méthoxycarbonylphényl)-pipéridine

A une suspension de 456 mg de Cs₂ CO₃ dans 2 ml de toluène anhydre on ajoute 3 mg (0,01 mmole) de Pd (OAc)₂, 10 mg (0,015 mmole), de 2,2'-bis(diphénylphosphino)-1,1'-binaphthyle (BINAP) 215 mg (1,2 mmole) de 4-bromo-1-méthoxycarbonylbenzène et 240 mg de produit de la Préparation 1 (1,2 mmole). On chauffe à 110° et après 2 jours on ajoute 2 ml de dioxane, 3 mg de Pd (OAC)₂ et 10 mg de BINAP. On chauffe encore pendant deux jours à 110° puis on arrête la réaction. On verse dans de l'eau et on extrait à l'acétate d'éthyle. Le produit est purifié par flash chromatographie en éluant par un mélange acétate d'éthyle/cyclohexane.

P.f. 162-165°C

### PREPARATION 7

### 4-(Phénylméthoxy)-3-(N-tert-butoxycarbonyl-N-méthansulfonylamino)-1-(2,3-époxypropoxy)-benzène et son isomère (2S).

Le produit du titre a été obtenu selon la procédure décrite dans WO 96/04233 (procédure 96).

### PREPARATION 8

### 4-Benzyloxy-3-méthylsulfinyl-1-[(2,3-époxypropoxy)]-benzène

Le produit du titre a été obtenu suivant la procédure décrite dans US 4,396,629

### PREPARATION 9

### 4-Benzyloxy-1-[(2,3-époxypropoxy)]-benzène

Le produit du titre a été obtenu suivant la procédure décrite dans WO 96/04233 (procédure 7)

### PREPARATION 10

### 4-tert-Butoxycarbonylamino-1-(4-hydroxycarbonylphenyl)-pipéridine

On dissout 2,19 g (0,0063 mole) du produit de la Préparation 4 dans 30 ml d'éthanol et 30 ml de THF et on y ajoute 20 ml d'eau et 12,6 ml (0,0126 mole) d'une solution de NaOH 1N. on agite le mélange à la température ambiante pendant 24 heures puis à 40 °C pendant 8 heures. On ajoute de l'acide acétique jusqu'à pH 5 et on évapore le solvant sous pression réduite. On reprend le résidu dans de l'eau, on filtre le solide et on le recristallise dans 200 ml d'éthanol. On obtient le produit du titre sous forme de solide blanc.

P.f. >300°C.

### PREPARATION 11

### Chlorhydrate de 4-amino-1-[4-(N-normal-butylaminocarbonyl)phenyl]-pipéridine hydraté

### 11a. 4-tert-Butoxycarbonylamino-1-[4-(N-normal-butylamino carbonyl)phenyl]-pipéridine

On dissout 2,5 g (0,0078 mole) du produit de la Préparation 10 dans 80 ml de chlorure de méthylène et on y ajoute 3,45 g (0,0078 mole) de BOP, 8 ml (0,0078 mole) de *normal*-butylamine et 1,7 ml (0,012 mole) de triéthylamine. On agite à 40°C pendant 8 heures, on évapore le solvant sous pression réduite, on reprend le résidu dans de l'acétate d'éthyle et on lave avec une solution saturée en bicarbonate de sodium. On filtre le solide formé et on le cristallise dans de l'isopropanol. On obtient le composé du titre sous forme de solide blanc.

P.f. 208-210°C.

### 11b. Chlorhydrate de 4-amino-1-[4-(N-nomral-butylaminocarbonyl)phenyl]-pipéridine hydraté

En opérant comme décrit dans la Préparation 5 mais en utilisant le produit de l'étape précédente au lieu du produit de la Préparation 4 on obtient le composé du titre.

P.f. 231-235°C (chlorhydrate hydraté).

### PREPARATION 12

### Dichlorhydrate de 4-amino-1-[4-(N,N-diéthylaminocarbonyl)phenyl]-pipéridine

### 12a. 4-tert-Butoxycarbonylamino-1-[4-(N,N-diéthylaminocarbonyl)phenyl]-pipéridine

En opérant comme décrit dans la Préparation 11a mais en utilisant la diéthylamine au lieu de la *normal*-butylamine, on obtient le composé du titre.

P.f. 113-115°C.

### 12b. Dichlorhydrate de 4-amino-1-[4-(N,N-diéthylaminocarbonyl)phenyl]-pipéridine

En opérant comme décrit dans la Préparation 11b mais en utilisant le produit de l'étape précédente au lieu du produit de la Préparation 11a on obtient le composé du titre.

P.f. 232-234°C (dichlorhydrate).

### PREPARATION 13

### 4-Benzyloxy-3-(N-tert-butoxycarbonyl-N-butansulfonyl-amino)-1-[((2S)-2,3-époxypropoxy)]-benzène

### 13a. Acétate de 4-benzyloxy-3-butansulfonylamino-benzène

On mélange sous atmosphère d'azote 5,0 g (0,00194 mole) d'acétate de 3-amino-4-benzyloxybenzène dans 15 ml de chlorure de méthylène et on y ajoute 3,3 ml de triéthylamine (0,0236 mole) et 3,3 ml (0,0245 mole) de 1-butansulfonylchlorure. On agite à la température ambiante pendant une nuit et après à 30°C pendant 4 heures. On lave à l'eau on sépare les deux phases on sèche la phase organique sur du sulfate de sodium on filtre et on évapore le solvant sous pression réduite. On purifie par chromatographie sur colonne de gel de silice en éluant par un mélange cyclohexane/acétate d'éthyle=9/1. On obtient le composé du titre.

P.f. 104-106°C

### 13b. Acétate de 4-benzyloxy-3-(N-tert-butoxycarbonyl-N-butansulfonyl-amino)-benzène

On mélange 3,4 g (0,009 mole) du produit de l'étape précédente dans 70 ml de chlorure de méthylène et on y ajoute 2,4 g (0,0108 mole) de di-tert-butyldicarbonate et 0,22 g (0,0018 mole) de 4-diméthylaminopyridine. On agite à la température ambiante pendant 3 heures, on évapore le solvant et on purifie par chromatographie sur colonne de gel de silice en éluant par un mélange cyclohexane/acétate d'éthyle=8/2. On obtient le composé du titre.

P.f. 74-76°C

### 13c. 4-Benzyloxy-3-(N-tert-butoxycarbonyl-N-butansulfonyl-amino)-phénol

On mélange 4 g (0,0083 mole) du produit de l'étape précédente dans 80 ml de méthanol et on y ajoute 9,9 g (0,0099 mole) de NaOH 1N. On agite à la température ambiante pendant 30 minutes on ajoute de l'acide citrique jusqu'à pH 6 et on évapore le solvant. On reprend à l'acétate d'éthyle on lave à l'eau on sépare les deux phases on sèche la phase organique sur du sulfate de sodium on filtre et on évapore le solvant sous pression réduite. On obtient le composé du titre.

P.f. 131-133°C.

### 13d. 4-Benzyloxy-3-(N-tert-butoxycarbonyl-N-butansulfonyl-amino)-1-[((2S)-2,3-époxypropoxy)]-benzène

On mélange 3,0 g (0,0062 mole) du produit de l'étape précédente dans 60 ml d'acétone et on y ajoute 2,8 g de carbonate de potassium anhydre râpé et 2,0 g (0,0077 mole) de (2S)(+)-glycidyl nosylate. On chauffe au reflux pendant 20 heures, on filtre, on évapore le solvant et on purifie par chromatographie sur colonne de gel de silice en éluant par un mélange cyclohexane/acétate d'éthyle=75 /25. On obtient le composé du titre.

P.f. 87-89°C

[a]_{D} = +4,9° (c : 1 % en methanol)

### PREPARATION 14

### 4-Benzylcarbonyloxy-3-(N-tert-butoxycarbonyl-N-propansulfonylamino)-1-[((2S)-2,3-époxypropoxy)]-benzène

En opérant comme décrit dans la Préparation 13 mais en utilisant le 1-propansulfonylchlorure au lieu du 1-butansulfonylchlorure on obtient le composé du titre.

[a]_{D} = +4,5° (c : 1% éthanol)

### PREPARATION 15

### 4-Benzyloxy-3-(N-methylamino-sulfonyl)-1-[((2S)-2,3-époxypropoxy)]-benzène 15a. 2,5-Dihydroxy-N-méthylbenzènesulfonamide

On agite à la température ambiante pendant 2 heures, 2,27 g (7,75 mmole) d'acétate de 4-acétyloxy-2-(chlorosulfonyl)phényle tel qu'obtenu suivant le procédé décrit dans J. Am. Chem Soc. 1951, 73 :2558-2565, dans 8 ml de tétrahydrofurane et 8 ml d'une solution de méthylamine (77,5 mmole) dans méthanol. On évapore le solvant et on reprend le résidu dans 10 ml d'eau acidifiée. On extrait à l'acétate d'éthyle, on sépare les deux phases on sèche la phase organique sur du sulfate de sodium on filtre et on évapore le solvant sous pression réduite. On purifie par chromatographie sur colonne de gel de silice en éluant par un mélange hexane/acétate d'éthyle=1/2. On obtient le composé du titre.

### 15b. 2-Hydroxy-5-[[tert-butyl(diméthyl)silyl]oxy]-N-méthylbenzènesulfonamide

On mélange 1,07 g (7,10 mmole) de chlorure de *tert*-butyldiméthylsilyle (TBDMSCI), 1,76 ml de 1,8-diazabicyclo[5.4.0]undec-7-ène (DBU) et on ajoute ce mélange à une solution de 1,4 g (6,9 mmole) du produit de l'étape précédente dans 20 ml de chlorure de méthylène et 5 ml de tétrahydrofurane. On agite à la température ambiante pendant 2 heures. On lave à l'eau et avec 20 ml d'une solution de H₃PO₄ à 5%et après avec une solution de bicarbonate de sodium. On sépare les deux phases on sèche la phase organique sur du sulfate de sodium on filtre et on évapore le solvant sous pression réduite. On obtient le composé du titre.

### 15c. Benzoate de 4-[[tert-butyl(diméthyl)silyl)oxy]-2-(N-methylamino-sulfonyl)-phényle

On mélange sous atmosphère d'azote 1,8 g (5,67 mmole) du produit de l'étape précédente dans 10 ml de chlorure de méthylène et on y ajoute 1,8 ml de pyridine et 1,3 ml de chlorure de benzoyle à 0°C.

on agite à la température ambiante pendant 3 heures puis on y ajoute une solution de bicarbonate de sodium. On extrait à l'acétate d'éthyle, on sépare les deux phases on sèche la phase organique sur du sulfate de sodium on filtre et on évapore le solvant sous pression réduite. On purifie par chromatographie sur colonne de gel de silice en éluant par un mélange hexane/acétate d'éthyle=2/1. On obtient le composé du titre.

### 15d. Benzoate de 4-hydroxy-2-(N-methylamino-suifonyl)-phényle

On mélange sous atmosphère d'azote 1,7 g (4,03 mmole) du produit de l'étape précédente dans 80 ml d'une solution d'acide chlorhydrique à 1% dans de l'éthanol 95%. On agite à la température ambiante pendant 3 heures. On y ajoute 100 ml d'eau, on extrait à l'acétate d'éthyle, on sépare les deux phases on sèche la phase organique sur du sulfate de sodium on filtre et on évapore le solvant sous pression réduite. On obtient le composé du titre.

P.f. 168-170°C.

### 15e. 4-Benzyloxy-3-(N-methylamino-sulfonyl)-1-[((2S)-2,3-époxypropoxy)]-benzène

En opérant comme décrit dans la Préparation 13d mais en utilisant le produit de l'étape précédente au lieu du produit de l'étape 13c, on obtient le compose du titre. [α]_{D} = + 19,9° (c= 1% en chloroforme)

### PREPARATION 16

### 4-tert-Butoxycarbonylamino-1-(4-cyanophényl)-pipéridine

En opérant comme décrit dans la Préparation 6 mais en utilisant le 4-bromocyanobenzène au lieu du 4-bromo-1-méthoxycarbonylbenzène, on obtient le compose du titre.

P.f. 188-189°C

### PREPARATION 17

### 4-Amino-1-(4-cyanophényl)-pipéridine

En opérant comme décrit dans la Préparation 5 mais en utilisant le produit de la Préparation 16 au lieu du produit de la Préparation 4. On libère la base avec de l'ammoniac et extraction avec acétate d'éthyle, on obtient le composé du titre.

P.f. 78-80°C.

### PREPARATION 18

### 4-tert-Butoxycarbonylaminométhyl-1-(4-éthoxycarbonylphényl)-pipéridine

En opérant comme décrit dans la Préparation 4 mais en utilisant la 4*-tert-*butoxycarbonylaminométhyl-pipéridine (décrite dans WO99/65895) au lieu de la 4-*tert-*butoxycarbonylamino-pipéridine de la Préparation 1, on obtient le compose du titre

### PREPARATION 19

### Chlorhydrate de 4-aminométhyl-1-(4-éthoxycarbonylphényl)-pipéridine

En opérant comme décrit dans la Préparation 5 mais en utilisant le produit de la Préparation 18 au lieu du produit de la Préparation 4, on obtient le composé du titre.

### PREPARATION 20

### 4-Benzyloxy-3-(N-benzyloxycarbonyl-N-méthansulfonylamino)-1-[((2S)-2,3-époxypropoxy)]-benzène

### 20a. Acétate de 4-benzyloxy-3-(N-benzyloxycarbonyl-N-méthansulfonylamino)-benzène

On agite à 50°C pendant 10 heures un mélange de 1 g (0.003 mole) d'acétate de 4-benzyloxy-3-méthansulfonylamino-benzène (WO 96/04233) dans 20 ml de chlorure de méthylène, 0,50 ml (0,0033 mole) de benzylchloroformiate à 95%, 0,006 g de diméthylaminopyridine et 0,46ml (0,0033 mole) de triéthylamine. On évapore le solvant on reprend du chlorure de méthylène, on lave à l'eau, on sépare les deux phases on sèche la phase organique sur du sulfate de sodium on filtre et on évapore le solvant sous pression réduite. On purifie par chromatographie sur colonne de gel de silice en éluant avec de l'acétate d'éthyle. On obtient le composé du titre.

P.f. 140-142°C.

### 20b. 4-Benzyloxy-3-(N-benzyloxycarbonyl-N-méthansulfonylamino)-phénol

En opérant comme décrit dans la Préparation 13c mais en utilisant le produit de l'étape précédente au lieu du produit de la Préparation 13b on obtient le composé du titre.

P.f.138-140°C.

### 20c. 4-Benzyloxy-3-(N-benzyloxycarbonyl-N-méthansulfonylamino)-1-[((2S)-2.3-époxypropoxy)]-benzène

En opérant comme décrit dans la Préparation 13d mais en utilisant le produit de l'étape précédente au lieu du produit de l'étape 13c, on obtient le compose du titre. P.f. 78-80°C ; [α]_{D} = + 4,4° (c= 0,5% en méthanol).

### PREPARATION 21

### 4-Amino-1-(4-tert-butoxycarbonylphenyl)-pipéridine

On mélange sous azote 1,5 g (7,5 mmole) de 4-(2,5-diméthylpyrrol-1-yl)-pipéridine, 60 ml de diméthylformamide et 1,6 g (7,5 mmole) d'ester *tert*-butylique de l'acide 4-fluoro-benzoique et 2,6 ml de diisopropyléthylamine. On chauffe sous agitation pendant 6 heures à 90°C et après on agite à la température ambiante pendant 15 heures. On y ajoute 1,04 g (7,5 mmole) de carbonate de potassium et on chauffe à 90°C pendant 5 jours. On verse dans de l'eau, on extrait à l'acétate d'éthyle et on lave avec de l'eau. On sépare les deux phases on sèche la phase organique sur du sulfate de sodium on filtre et on évapore le solvant sous pression réduite. On purifie par chromatographie sur colonne de gel de silice en éluant par un mélange hexane/acétate d'éthyle=3/1. On isole le 4-(2,5-diméthylpyrrol-1-yl)-1-(4-*tert-*butoxycarbonylphenyl)-pipéridine. On mélange sous azote 250 mg de NH₂OH.HCl et 0,7 ml d'éthanol et on agite pendant 30 minutes. On y ajoute 67 mg (1,2 mmole) de KOH dans 0,3 ml d'une solution d'éthanol/eau 1/1 et 300 mg (0,084 mmole) du produit intermédiaire ci-dessus dans 0,5 ml d'éthanol. On chauffe à 80°C pendant 12 heures. On extrait à l'acétate d'éthyle, on ajoute à la phase aqueuse du KOH jusqu'à pH 9 et on extrait à l'acétate d'éthyle. On sépare les deux phases on sèche la phase organique sur du sulfate de sodium on filtre et on évapore le solvant sous pression réduite. On obtient le composé du titre.

P.f. 89-91°C.

### PREPARATION 22

### 4-tert-Butoxycarbonylamino-1-(4-aminocarbonylphényl)-pipéridine

En opérant comme décrit dans la Préparation 4 mais en utilisant le 4-fluoro-benzamide au lieu du 4-éthoxycarbonyl-1-fluoro-benzène, on obtient le composé du titre.

P.f. >270°C.

### PREPARATION 23

### Chlorhydrate de 4-amino-1-(4-aminocarbonylphényl)-pipéridine

En opérant comme décrit dans la Préparation 5 mais en utilisant le produit de la Préparation 22 au lieu du produit de la Préparation 4, on obtient le composé du titre.

### EXEMPLE 1

### 3-[1-(4-Ethoxycarbonylphénylméthyl)-pipéridin-4-ylamino]-1-(4-hydroxyphénoxy)-2-propanol

### 1a. 3-[1-(4-Ethoxycarbonylphénylméthyl)-pipéridin-4-ylamino]-1-(4-méthoxy-éthoxy-méthoxyphenoxy)-2-propanol

On chauffe au reflux pendant 17 heures 0,86 g (0,0033 mole) de 4-(méthoxy-éthoxy-méthoxy)-1-(2,3-époxypropoxy)-benzène et 0,83 g (0,0033 mole) du produit obtenu dans la Préparation 3 sous forme de base libre dans 40 ml d'éthanol. On évapore le solvant sous pression réduite et on sèche le produit. On purifie le brut de réaction par flash-chromatographie en éluant par méthanol. Le produit est cristallisé en éther isopropylique.

P.f. 73-75°C

### 1b. Dioxalate de 3-[1-(4-éthoxycarbonylphénylméthyl)-pipéridin-4-ylamino]-1-(4-hydroxyphénoxy)-2-propanol

On chauffe à 40°C pendant 8 heures un mélange contenant 0,7 g (0,0014 mole) du produit obtenu dans l'étape précédente et 1,1ml (0,014 mole) de CF₃COOH dans 40 ml de chlorure de méthylène. On évapore le solvant et on ajoute de l'ammoniac. On extrait à l'acétate d'éthyle, on anhydrifie et on évapore le solvant. Le brut de réaction est purifié par flash-chromatographie en éluant par chlorure de méthylène/méthanol =9 : 1. On obtient le composé du titre sous forme de base. On prépare son dioxalate à l'aide d'acide oxalique dans de l'acétone.

P.f. 180-184°C (dioxalate)

### EXEMPLE 2

### 3-[1-(4-Ethoxycarbonylphényl)-pipéridin-4-ylamino]-1-(4-hydroxyphénoxy)-2-propanol

### 2a. 3-[1-(4-Ethoxycarbonylphényl)-pipéridinylamino]-1-(4-benzyloxyphenoxy)-2-propanol

On chauffe au reflux pendant 20 heures 1,03 g (0,004 mole) du produit de la Préparation 9 et 1 g (0,004 mole) du produit de la Préparation 5 sous forme de base dans 50 ml d'éthanol. On évapore le solvant et on purifie le brut de réaction par flash-chromatographie en éluant par un mélange CH₂Cl₂/méthanol = 9:1.

On obtient le produit du titre.

P.f. 112-114°C

### 2b. 3-[1-(4-Ethoxycarbonylphénylméthyl)-pipéridin-4-ylamino]-1-(4-hydroxyphenoxy)-2-propanol

On hydrogène à 40 °C à la pression ambiante pendant huit heures 1,15 g (0,0023 mole) du produit de l'étape précédente dans 20 ml d'éthanol + 20 ml de THF en présence de 0,1 g de palladium sur carbone à 10 %. 150 ml d'hydrogène sont absorbés.

On filtre et on évapore le solvant. On cristallise dans de l'acétate d'éthyle. On obtient le produit du titre.

P.f. 158-161°C

### EXEMPLE 3

### 3-[1-(4-Ethoxycarbonylphényl)-pipéridin-4-ylamino]-1-[(4-hydroxy)-3-(méthansulfonylamino)-phénoxy]-2-propanol et son isomère (2S)

### 3a. 3-[1-(4-Ethoxycarbonylphényl)-pipéridin-4-ylamino]-1-[(4-benzyloxy)-3-(méthansulfonylamino)-phénoxy]-2-propanol

On mélange 1 g (0,004 mole) du produit de la Préparation 5 sous forme de base avec 1,35 g (0,003 mole) du produit de la Préparation 7 et 0,2 g de perchlorate de lithium dans 50 ml de CH₃CN. On laisse sous agitation pendant 24 heures à la température ambiante, puis on chauffe à 40° pendant huit heures.

On évapore le solvant et le produit ainsi obtenu est traité à 40° pendant huit heures avec une solution d'acide chlorhydrique dans de l'acétate d'éthyle. On évapore le solvant on traite avec une solution de NaHCO₃, on extrait à l'acétate d'éthyle. On évapore encore le solvant et on purifie le brut de réaction par flash-chromatographie en éluant par un mélange CH₂Cl₂-méthanol = 9:1. On obtient le produit du titre.

P.f. 130-132°C..

### 3b. 3-[1-(4-Ethoxycarbonylphényl)-pipéridin-4-ylamino]-1-[(4-hydroxy)-3-(méthansulfonylamino)-phénoxy]-2-propanol

0,8 g (0,0013 mole) du produit de l'étape précédente sont soumis à hydrogénation en présence de 0,1 g de palladium sur carbone (10%) dans 15 ml d'éthanol + 15 ml de THF. Après huit heures de réaction à 40° et à la pression ambiante, on filtre et on évapore le solvant. On purifie le brut de réaction par flash-chromatographie en éluant par un mélange CH₂Cl₂/méthanol = 9:1. On obtient le produit du titre qui cristallise dans l'isopropanol.

P.f. 140-143°C.

### Isomère (2S)

En opérant selon les étapes 3a et 3b ci-dessus, mais en utilisant le produit de la

Préparation 7 sous forme optiquement active (2S), on obtient l'énantiomère (2S) du composé du titre.

P.f. 96-99°C (forme hydraté).

### EXEMPLE 4

### 3-[1-(4-Ethoxycarbonylphényl)-pipéridin-4-ylamino]-1-[(4-hydroxy)-3-(méthysulphinyl)-phénoxy]-2-propanol et son isomère (2S)

### 4a. 3-[1-(4-Ethoxycarbonylphényl)-pipéridin-4-ylamino]-1-[(4-benzyloxy)-3-(méthysulphinyl)-phénoxy]-2-propanol

On chauffe au reflux pendant une nuit 0,8 g (0,0032 mole) du produit de la Préparation 5 sous forme de base avec 1 g (0,0031 mole) du produit de la Préparation 8 dans 50 ml d'éthanol. On évapore le solvant et on purifie le brut de réaction par flash-chromatographie en éluant par un mélange CH₂Cl₂/méthanol = 95:5. On obtient le produit du titre.

P.f. 135-137°C.

### 4b. Trifluoroacétate de 3-[1-(4-éthoxycarbonylphényl)-pipéridin-4-ylamino]-1-[(4-hydroxy)-3-(méthysulphinyl)-phénoxy]-2-propanol

On chauffe à 55°C pendant sept heures 0,98 g (0,0017 mole) du produit de l'étape précédente dans 20 ml de CF₃COOH. On évapore le solvant, on ajoute une solution de bicarbonate et on extrait à l'acétate d'éthyle. On évapore le solvant et on purifie le brut de réaction par flash-chromatographie en éluant par un mélange CH₂-Cl₂/méthanol = 9:1. On obtient le produit du titre.

P.f. 78-80°C (trifluoroacétate).

### EXEMPLE 5

### 3-[1-(4-N-Butylaminocarbonylphényl)-pipéridinylamino]-1-[(4-hydroxy)-3-(méthansulfonylamino)-phénoxy]-(2S)-2-propanol

### 5a. 3-[1-(4-N-Butylaminocarbonylphényl)-pipéridinylamino]-1-[(4-benzyloxy)-3-(méthansulfonylamino)-phénoxy]-(2S)-2-propanol

On chauffe au reflux pendant une nuit 0,72 g (0,0026 mole) du produit de la Préparation 11b sous forme de base avec 1,08 g (0,0024 mole) du produit de la Préparation 7 dans 25 ml d'éthanol. On évapore le solvant et on purifie le brut de réaction par flash-chromatographie en éluant par du méthanol. Le produit ainsi obtenu est traité à 70° pendant 4 heures avec une solution d'acide chlorhydrique dans de l'acétate d'éthyle. On évapore le solvant on traite avec une solution de NaHCO₃, on extrait à l'acétate d'éthyle. On évapore encore le solvant. On obtient le produit du titre.

P.f. 123-133°C.

### 5b. 3-[1-(4-N-Butylaminocarbonylphényl)-pipéridin-4-ylamino]-1-[(4-hydroxy)-3-(méthansulfonylamino)-phénoxy]-(2S)-2-propanol

En opérant comme décrit dans l'Exemple 3b mais en utilisant le produit de l'étape précédente au lieu du produit de l'étape 3a, on obtient le composé du titre.

P.f. 146-148°C.

### EXEMPLE 6

### 3-[1-(4-N,N-Diéthylminocarbonylphényl)-pipéridiny-4-yl-amino]-1-[(4-hydroxy)-3-(méthansulfonylamino)-phénoxy] ]-(2S)-2-propanol

En opérant comme décrit dans l'Exemple 5 mais en utilisant le produit de la Préparation 12b sous forme de base au lieu du le produit de la Préparation 11b on obtient le composé du titre.

P.f. 67-70°C.

### EXEMPLE 7

### 3-[1-(4-Ethoxycarbonylphényl)-pipéridin-4-ylamino]-1-[(4-hydroxy)-3-(N-butansulfonylamino)-phénoxy]-(2S)-2-propanol et son chlorhydrate

En opérant comme décrit dans l'Exemple 5 mais en utilisant le produit de la Préparation 13d au lieu du le produit de la Préparation 7 et le produit de la Préparation 5 sous forme de base au lieu du le produit de la Préparation 11b, on obtient le composé du titre. On prépare le chlorhydrate à l'aide d'acétate d'éthyle et acide chlorhydrique.

P.f. 192-195°C (chlorhydrate).

### EXEMPLE 8

### 3-[1-(4-Ethoxycarbonylphényl)-pipéridin-4-ylamino]-1-[(4-hydroxy)-3-(N-propansulfonylamino)-phénoxy]-(2S)-2-propanol

En opérant comme décrit dans l'Exemple 5 mais en utilisant le produit de la Préparation 14 au lieu du le produit de la Préparation 7 et le produit de la Préparation 5 sous forme de base au lieu du le produit de la Préparation 11 b, on obtient le composé du titre.

P.f. 63-65°C.

### EXEMPLE 9

### 3-[1-(4-Ethoxycarbonylphényl)-pipéridin-4-ylamino]-1-[(4-hydroxy)-3-(méthylamino-sulfonyl)-phénoxy]-(2S)-2-propanol

On chauffe au reflux pendant une nuit 0,56 g (0,0015 mole) du produit de la

Préparation 15e avec 0,38 g (0,0015 mole) du produit de la Préparation 5 sous forme de base dans 10 ml de DMF. On évapore le solvant et on purifie le brut de réaction par flash-chromatographie en éluant par du méthanol. On obtient le produit du titre.

P.f. 87°C

### EXEMPLE 10

### 3-[1-(4-Cyanophényl)-pipéridin-4-ylamino]-1-[(4-hydroxy)-3-(N-méthansulfonylamino)-phénoxy]-(2S)-2-propanol

En opérant comme décrit dans l'Exemple 5 mais en utilisant le produit de la

Préparation 17 au lieu du produit de la Préparation 11b, on obtient le composé du titre.

P.f. 78-80°C.

### EXEMPLE 11

### 3-[1-(4-tert-Butoxycarbonylphényl)-pipéridin-4-ylamino]-1-[(4-hydroxy)-3-(N-méthansulfonylamino)-phénoxy]-(2S)-2-propanol

En opérant comme décrit dans l'Exemple 3 mais en utilisant le produit de la Préparation 21 au lieu du produit de la Préparation 5 et le produit de la Préparation 20 au lieu du produit de la Préparation 7, on obtient le composé du titre.

### EXEMPLE 12

### 3-[[1-(4-Ethoxycarbonylphényl)-4-pipéridinyl-méthyl]amino]-1-[(4-hydroxy)-3-(méthansulfonylamino)-phénoxy]-(2S)-2-propanol

En opérant comme décrit dans l'Exemple 5 mais en utilisant le produit de la Préparation 19 au lieu du produit de la Préparation 11b, on obtient le composé du titre.

### EXEMPLE 13

### 5-[((4-Isopropylphényl)sulfonyl)amino]-2-(4-((3-(4-hydroxyphénoxy)-2-hydroxypropyl)amino)pipéridino)-pyridine

### 13a. 5-Amino-2-(4-tert-butoxycarbonylaminopipéridino)-pyridine

On mélange 2 g (0,0062 mole) de 5-nitro-2-(4-*tert*-butoxycarbonylpipéridino)-pyridine dans 40 ml d'éthanol et 60 ml de tétrahydrofurane. On ajoute 0,4g de Pd/C à 10% et on hydrogène à 40°C, à la pression ambiante pendant 7 heures. On filtre, on évapore le solvant et on obtient 2 g du composé du titre sous forme de solide.

### 13b. 5-[((4-Isopropylphényl)sulfonyl)amino]-2-(4-tert-butoxycarbonylamino pipéridino)-pyridine

On dissout 0,4 g (0,0013 mole) du produit de l'étape précédente dans 10 ml de pyridine. On y ajoute 0,3 g (0,0013 mole) de chlorure de 4-isopropylbenzènesulfonyle et on chauffe à 50°C pendant 2 heures. On évapore le solvant (avec les précautions pour l'acide chlorhydrique), on reprend le résidu dans de l'acétate d'éthyle et de l'eau. On sépare les deux phases, on lave la phase organique à l'eau, on sèche et on évapore le solvant sous pression réduite. On purifie par flash-chromatographie sur colonne de gel de silice en éluant par un mélange cyclohexane/acétate d'éthyle = 6/4. On obtient le produit du titre.

P.f. 208-209°C.

### 13c. 5-[((4-Isopropylphényl)sulfonyl)amino]-2-(4-aminopipéridino)-pyridine dichlorhydrate

On chauffe au reflux pendant 4 heures 2,1 g (0,0042 mole) du produit de l'étape précédente dans 20 ml d'acétate d'éthyle et 20 ml d'une solution environ 3N d'acide chlorhydrique gazeux en acétate d'éthyle. On évapore le solvant sous pression réduite, on reprend le résidu dans de l'acétone, on filtre, on lave le précipité à l'acétone et on obtient 1,8 g du produit du titre qui est cristallisé dans de l'éthanol.

P.f. 270-273 °C.

### 13d. 5-[((4-Isopropylphényl)sulfonyl)amino]-2-(4-((3-(4-(benzyloxy)phénoxy)-2-hydroxypropyl)amino)pipéridino)-pyridine

On chauffe au reflux pendant 20 heures 0,239 g (0,932 mole) de 4-benzyloxy-1-(2,3-époxypropoxy)benzène, 0,35g (0,935 mmole) du produit de l'étape précédente sous forme de base dans 10 ml d'éthanol. On évapore le solvant sous pression réduite et on purifie par flash-chromatographie sur colonne de gel de silice en éluant par un mélange chlorure de méthylène/méthanol = 95/5. On obtient 0,37 g du produit du titre sous forme de produit vitreux.

### 13^{e}. 5-[((4-Isopropylphényl)sulfonyl)amino]-2-(4-((3-(4-hydroxyphénoxy)-2-hydroxypropyl)amino)pipéridino)-pyridine

On mélange 0,37 g (0,586 mmole) du produit de l'étape précédente, 10 ml d'éthanol, 10 ml de tétrahydrofurane et 0,037 g de Pd/C à 10%. On hydrogène à 40°C et pression ambiante pendant 8 heures. On filtre, on évapore le solvant sous pression réduite et on purifie par flash-chromatographie sur colonne de gel de silice en éluant par un mélange chlorure de méthylène/méthanol 9/1. On obtient le produit du titre. P.f. 75-78°C.

### EXEMPLE 14

### 14a. 5-[((4-Isopropylphényl)sulfonyl)amino]-2-(4-((3-(4-(benzyloxy)-3-(méthylsulfinyl)phénoxy)-2-hydroxypropyl)amino)pipéridino)-pyridine

On chauffe au reflux pendant une nuit 0.27 g (0,00084 mole) de 4-benzyloxy-3-méthylsulfinyl-1-[(2,3-époxypropoxy)]-benzène (préparé selon US 4,396,629/exemple 3) et 0.33g (0,00088 mole) du produit de l'exemple 13c) sous forme de base dans 10 ml de éthanol. On évapore le solvant et on purifie le brut de réaction par flash-chromatographie en éluant par un mélange CH₂Cl₂/méthanol = 9/l. On obtient le produit du titre sous forme de solide vitreux.

### 14b. 5-[((4-Isopropylphényl)sulfonyl)amino]-2-(4-((3-(4-hydroxy-3-(méthylsulfinyl)phénoxy)-2-hydroxypropyl)amino)pipéridino)-pyridine

On chauffe pendant 5 heures à 55°C 0,4g (0,0006 mole) du produit de l'étape précédente dans 10 ml de CF₃COOH. On évapore le solvant sous pression réduite, on dissout le brut dans de l'acétate d'éthyle, on lave à l'aide d'une solution aqueuse saturé en bicarbonate et on sèche. Le produit ainsi obtenu est purifié par flash-chromatographie en éluant par un mélange CH₂Cl₂/ méthanol = 9/1 et puis par un mélange CH₂Cl₂/ méthanol = 85/15. On obtient le produit du titre.

P.f.: 128-130°C.

### EXEMPLE 15

### S-[((4-Isopropylphényl)sulfonyl)amino]-2-(4-((3-(4-hydroxy-3-méthansulfonylamino)phénoxy)-(2S)-2-hydroxypropyl)amino)pipéridino)-pyridine

En opérant comme décrit dans l'Exemple 5 mais en utilisant le produit de l'Exemple 13c sous forme de base au lieu du produit de la Préparation 11b, on obtient le composé du titre.

### EXEMPLE 16

### 5-[((4-Bromophényl)sulfonyl)amino]-2-(4-((3-(4-hydroxy-3-(méthylsulfinyl)phénoxy)-2-hydroxypropyl)amino)pipéridino)-pyridine

### 16a. 5-[((4-Bromophényl)sulfonyl)amino]-2-[4-(tert-butoxycarbonyl-amino)-pipéridino]-pyridine

En opérant comme dans l'Exemple 13b mais en utilisant le chlorure de 4-bromobenzènesulfonyle au lieu du chlorure de 4-isopropylbenzènesulfonyle, on obtient le composé du titre.

### 16b. 5-[((4-Bromophényl)sulfonyl)amino]-2-(4-((3-(4-hydroxy-3-(méthylsulfinyl)phénoxy)-2-hydroxypropyl)amino)pipéridino)-pyridine

En opérant comme dans l'Exemple 13c on déprotège le produit de l'étape précédente. On chauffe au reflux pendant 12 heures 1,2 g ce produit, 1,12 ml de triéthylamine, 0,738 g de 4-benzyloxy-3-méthylsulfinyl-1-[(2,3-époxypropoxy)]-benzène (préparé selon US 4,396,629/exemple 3) dans 100 ml d'éthanol. On évapore le solvant, et on purifie par flash-chromatographie en éluant par un mélange CH₂Cl₂/ méthanol/NH₃ = 90/10/1. On chauffe à 55°C pendant 5 heures un mélange contenant 680 mg du produit ainsi obtenu et 30 ml de CF₃COOH. On évapore le solvant et on traite avec une solution saturée en bicarbonate de sodium. On extrait à l'acétate d'éthyle, on sèche et on évapore le solvant. Le brut de réaction est purifié par flash-chromatographie en éluant par chlorure de méthylène/méthanol/NH₃ =90/10/1. On obtient le composé du titre sous forme de base.

P.f. 147°C.

### EXEMPLE 17

### 3-[1-(4-Ethoxycarbonylphényl)-pipéridin-4-ylamino]-1-(4-hydroxy-3-méthylsulfonyl-phénoxy)-(2S)-2-propanol

En opérant comme décrit dans l'Exemple 14 mais en utilisant le 4-benzyloxy-3-méthylsulfonyl-1-((2S)-2,3-époxypropoxy)-benzène (décrit dans WO99/65895, ex.67) au lieu du 4-benzyloxy-3-méthylsulfinyl-1-[(2,3-époxypropoxy)]-benzène et le produit de la Préparation 5 sous forme de base au lieu du produit de l'exemple 13c) on obtient le composé du titre.

P.f. 83-85°C ; [α]_{D} = + 1,0° (c= 1% en méthanol).

### EXEMPLE 18

### 3-[1-[4-((4-Isopropylphényl)-sulfonylamino)phényl]-pipéridinylamino]-1-(4-hydroxy-3-méthansulfonylamino-phénoxy)-(2S)-2-propanol

En opérant comme décrit dans l'Exemple 5 mais en utilisant la 4-amino-1-[4-((4-isopropylphényl)-sulfonylamino)-phényl]-pipéridine au lieu du produit de la Préparation 11b on obtient le composé du titre.

P.f. 90-93°C.

### EXEMPLE 19

### 3-[1-[4-((4-Bromophényl)-sulfonylamino)-phényl]-pipéridinylamino]-1-(4-hydroxy-3-méthansulfonylamino-phénoxy)-(2S)-2-propanol

On chauffe au reflux pendant 12 heures 0,95 g (0,0023 mole) de 4-amino-1-[4-((4-bromophényl)-sulfonylamino)-phényl]-pipéridine et 1,1 g (0,0024 mole) du produit de la Préparation 7 isomère (2S) dans 100 ml d'éthanol. On évapore le solvant, et on purifie par flash-chromatographie en éluant par un mélange CH₂Cl₂/ méthanol = 80/20. On chauffe à 55°C pendant 5 heures un mélange contenant 680 mg du produit ainsi obtenu et 30 ml de CF₃COOH. On évapore le solvant et on traite avec une solution saturée en bicarbonate de sodium. On extrait à l'acétate d'éthyle, on sèche et on évapore le solvant. Le brut de réaction est purifié par flash-chromatographie en éluant par chlorure de méthylène/méthanol =90/10. On obtient le composé du titre.

P.f. 105-108°C.

## Revendications

1. Composés de formule (I) 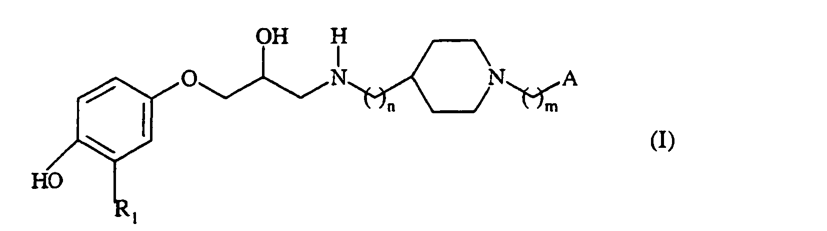 où
R₁ représente un atome d'hydrogène ou d'halogène, un groupe -S(O)_{Z}-(C₁-C₄)alkyle, -S(O)_{Z}-(C₁-C₄)R₃, -SO₂-NH-(C₁-C₄)alkyle, -NHCO(C₁-C₄)alkyle, -CO(C₁-C₄)alkyle ou-NHSO₂-(C₁-C₄)alkyle ;
m et n représentent indépendamment 0, 1 ou 2 ;
A représente un groupe de formule (a) ou (b) : 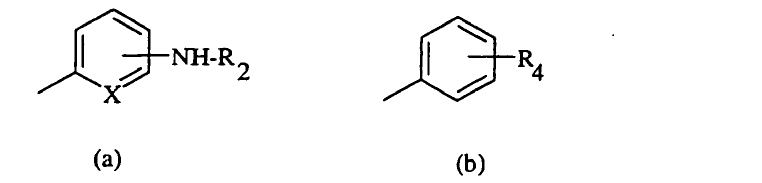
où
X est N ou CH ;
R₂ représente un groupe -SO₂-R₃, -CO-R₃ ou -CO-(C₁-C₄)alkyle ;
R₃ représente un groupe phényle, éventuellement substitué par un groupe (C₁-C₄)alkyle, (C₁-C₄)alkoxy, un ou deux atomes d'halogène ou un hétérocycle;
R₄ représente un atome d'hydrogène ou d'halogène, un groupe (C₁-C₆)alkyle, (C₁-C₄)alcoxy, -COOH, -COO(C₁-C₄)alkyle, -CN, -CONR₅R₆, -NO₂, -NHSO₂(C₁-C₄)alkyle ou - SO₂NR₅ R₆;
z est 1 ou 2;
R₅ et R₆ représentent indépendamment un atome d'hydrogène ou un groupe (C₁-C₄)alkyle, phényle ou phényl(C₁-C₄)alkyle ;
et leurs sels ou solvates.

2. Composés selon la revendication 1 où A est un groupe (a), X est N et le groupe NHR₂ est dans la position 5 de la pyridine.

3. Composés selon la revendication 1 où n et m sont zéro.

4. Composés selon la revendication 1 où le groupe (C₁-C₄)alkyle est un groupe méthyle ou éthyle.

5. Composés selon la revendication 1 où A est un groupe (b) et le groupe R₄ est dans la position 4 du benzène.

6. Composés selon la revendication 1 où A est un groupe (b) et R₄ est choisi parmi -COOH, -COO(C₁-C₄)alkyle, -CN, -NO₂, -CONR₂R₃, -NHSO₂-(C₁-C₄)alkyle et -SO₂NR₅R₆.

7. Procédé pour la préparation des composés de formule (I) **caractérisé en ce qu'**on fait réagir un composé de formule (II) 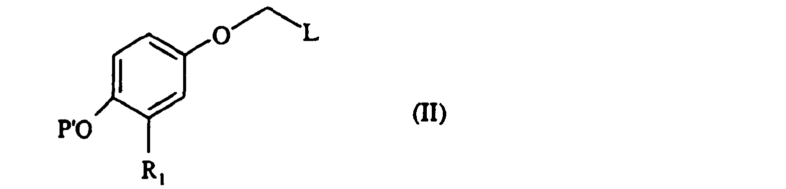 dans laquelle R₁ est tel qu'indiqué dans la revendication 1, P' est un groupe protecteur et L est un groupe de formule (c) ou (d) 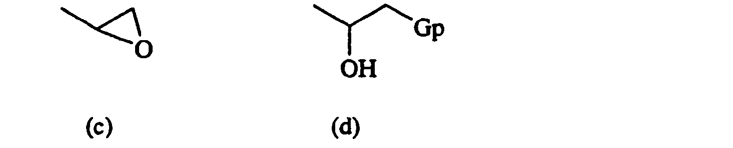 où Gp est un groupe partant, avec une amine de formule (III) 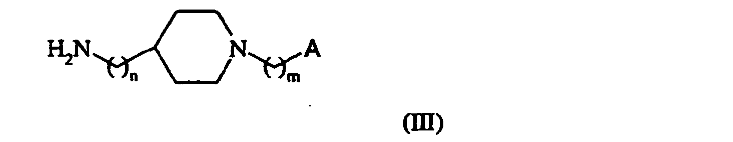 dans laquelle n et m sont tels que définis dans la revendication 1, en clivant le groupe P' et éventuellement en transformant le composé de formule (I) ainsi obtenu en l'un de ses sels.

8. Composition pharmaceutique contenant en tant que principe actif, un composé selon les revendications 1 à 6.

9. Utilisation d'un composé selon les revendications 1 à 6 pour la préparation de médicaments indiqués dans le syndrome du colon irritable (IBD), ou à action modulatrice de la motricité intestinale, lipolytique, anti-obésité, anti-diabétique, psychotrope, anti-glaucomateuse, cicatrisante, anti-dépressante, tocolytique.

10. Composés de formule (III') 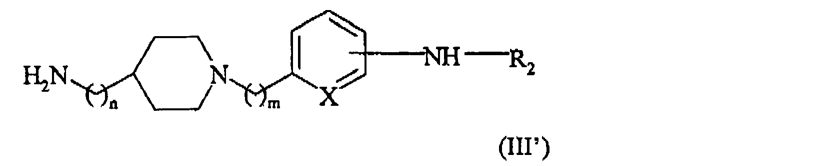 où n, m, X et R₂ sont tels que définis dans la revendication 1, ainsi que leurs sels ou solvates.

11. Composés de formule (III") 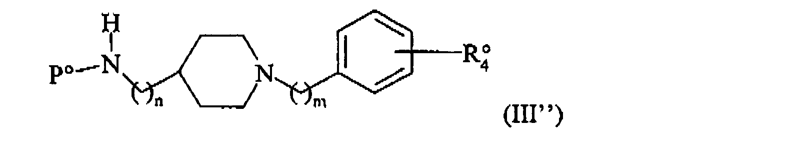 où
P^{o} est un groupe *tert*-butoxycarbonyle;
n et m sont 0, 1 ou 2;
R^{o} ₄ est un groupe choisi parmi -COO(C₁-C₄)alkyle, -CONR^{o} ₅R^{o} ₆ et -NHSO₂(C₁-C₄)alkyle;
R^{o} ₅ et R^{o} ₆ représentent indépendamment un atome d'hydrogène ou un groupe (C₁-C₄)alkyle ;
ainsi que leurs sels ou solvates.

12. Composés de formule (I) selon la revendication 1, **caractérisé en ce qu'**il consiste en le :
• 3-[1-(4-Ethoxycarbonylphénylméthyl)-pipéridin-4-ylamino]-1-(4-hydroxyphénoxy)-2-propanol
• 3-[1-(4-Ethoxycarbonylphényl)-pipéridin,-4-ylamino]-1-(4-hydroxyphénoxy)-2-propanol
• 3-[1-(4-Ethoxycarbonylphényl)-pipéridin-4-ylamino]-1-[(4-hydroxy)-3-(méthansulfonylamino)-phénoxy]-2-propanol
• 3-[1-(4-Ethoxycarbonylphényl)-pipéridin-4-ylamino]-1-[(4-hydroxy)-3-(méthysulphinyl)-phénoxy]-2-propanol
• 3-[1-(4-N-Butylaminocarbonylphényl)-pipéridinylamino]-1-[(4-hydroxy)-3-(méthansulfonylamino)-phénoxy]-(2S)-2-propanol
• 3-[1-(4-N,N-diéthylminocarbonylphényl)-pipéridiny-4-yl-amino]-1-[(4-hydroxy)-3-(méthansulfonylamino)-phénoxy]]-(2S)-2-propanol
• 3-[1-(4-Ethoxycarbonylphényl)-pipéridin-4-ylamino]-1-[(4-hydroxy)-3-(N-butansulfonylamino)-phénoxy]-(2S)-2-propano
• 3-[1-(4-Ethoxycarbonylphényl)-pipéridin-4-ylamino]-1-[(4-hydroxy)-3-(N-propansulfonylamino)-phénoxy]-(2S)-2-propanol
• 3-[1-(4-Ethoxycarbonylphényl)-pipéridin-4-ylamino]-1-[(4-hydroxy)-3-(méthylamino-sulfonyl)-phénoxy]-(2S)-2-propanol
• 3-[1-(4-Cyanophényl)-pipéridin-4-ylamino]-1-[(4-hydroxy)-3-(N-méthansulfonylamino)-phénoxy]-(2S)-2-propanol
• 3-[1-(4-*tert*-Butoxycarbonylphényl)-pipéridin-4-ylamino]-1-[(4-hydroxy)-3-(N-méthansulfonylamino)-phénoxy]-(2S)-2-propanol
• 3-[[1-(4-Ethoxycarbonylphényl)-4-pipéridinyl-méthyl]amino]-1-[(4-hydroxy)-3-(méthansulfonylamino)-phénoxy]-(2S)-2-propanol
• 5-[((4-Isopropylphényl)sulfonyl)amino]-2-(4-((3-(4-hydroxyphénoxy)-2-hydroxypropyl)amino)pipéridino)-pyridine
• 5-[((4-Isopropylphényl)sulfonyl)amino]-2-(4-((3-(4-(benzyloxy)-3-(méthylsulfinyl)phénoxy)-2-hydroxypropyl)amino)pipéridino)-pyridine
• 5-[((4-Isopropylphényl)sulfonyl)amino]-2-(4-((3-(4-hydroxy-3-méthansulfonylamino)phénoxy)-(2S)-2-hydroxypropyl)amino)pipéridino)-pyridine
• 5-[((4-Bromophényl)sulfonyl)amino]-2-(4-((3-(4-hydroxy-3-(méthylsulfinyl)phénoxy)-2-hydroxypropyl)amino)pipéridino)-pyridine
• 3-[1-(4-Ethoxycarbonylphényl)-pipéridin-4-ylamino]-1-(4-hydroxy-3-méthylsulfonyl-phénoxy)-(2S)-2-propanol
• 3-[1-[4-((4-Isopropylphényl)-sulfonylamino)phényl]-pipéridinylamino]-1-(4-hydroxy-3-méthansulfonylamino-phénoxy)-(2S)-2-propanol
ou
• 3-[1-[4-((4-Bromophényl)-sulfonylamino)-phényl]-pipéridinylamino]-1-(4-hydroxy-3-méthansulfonylamino-phénoxy)-(2S)-2-propanol et ses sels ou solvates

## Claims

1. Compounds of formula (I) 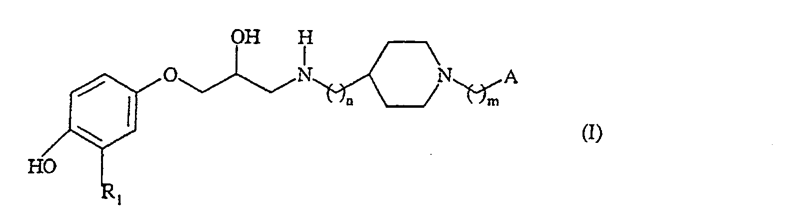 where
R₁ represents a hydrogen or halogen atom or an -S(O)_{z}-(C₁-C₄)alkyl, -S(O)_{z}-(C₁-C₄)R₃, -SO₂-NH- (C₁-C₄)alkyl, -NHCO (C₁-C₄) alkyl, -CO (C₁-C₄) alkyl or -NHSO₂- (C₁-C₄) alkyl group;
m and n independently represent 0, 1 or 2;
A represents a group of formula (a) or (b) : 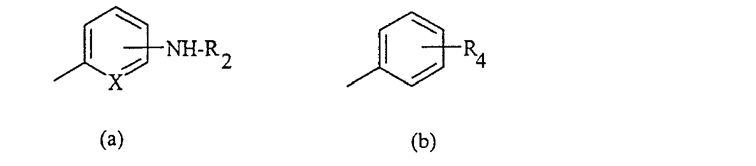
where
X is N or CH;
R₂ represents an -SO₂-R₃, -CO-R₃ or -CO-(C₁-C₄)alkyl group;
R₃ represents a phenyl group, optionally substituted by a (C₁-C₄) alkyl or (C₁-C₄) alkoxy group, one or two halogen atoms or a heterocycle;
R₄ represents a hydrogen or halogen atom or a (C₁-C₆) alkyl, (C₁-C₄)alkoxy, -COOH, -COO (C₁-C₄) alkyl, -CN, -CONR₅R₆, -NO₂, -NHSO₂(C₁-C₄)alkyl or -SO₂NR₅R₆ group;
z is 1 or 2;
R₅ and R₆ independently represent a hydrogen atom or a (C₁-C₄)alkyl, phenyl or phenyl (C₁-C₄) alkyl group;
and their salts or solvates.

2. Compounds according to Claim 1, where A is a group (a), X is N and the NHR₂ group is in the 5 position of the pyridine.

3. Compounds according to Claim 1, where n and m are zero.

4. Compounds according to claim 1, where the (C₁-C₄)alkyl group is a methyl or ethyl group.

5. Compounds according to Claim 1, where A is a group (b) and the R₄ group is in the 4 position of the benzene.

6. Compounds according to Claim 1, where A is a group (b) and R₄ is chosen from -COOH, -COO (C₁-C₄) - alkyl, -CN, -NO₂, -CONR₂R₃, -NHSO₂-(C₁-C₄)alkyl and -SO₂NR₅R₆.

7. Process for the preparation of the compounds of formula (I), **characterized in that** a compound of formula (II) 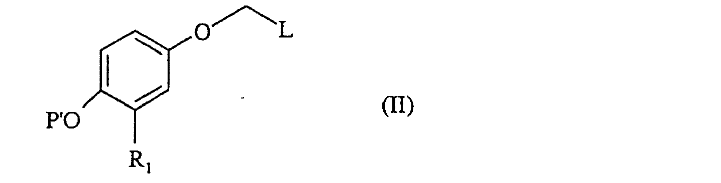 in which R₁ is as indicated in Claim 1, P' is a protective group and L is a group of formula (c) or (d) 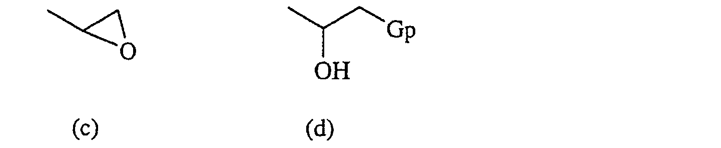 where Gp is a leaving group, is reacted with an amine of formula (III) 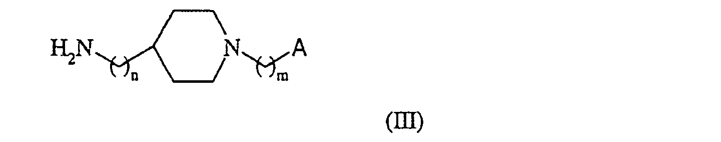 in which n and m are as defined in Claim 1, the group P' being cleaved and the compound of formula (I) thus obtained optionally being converted into one of its salts.

8. Pharmaceutical composition comprising, as active principle, the compound according to Claims 1 to 6.

9. Use of the compound according to Claims 1 to 6 for the preparation of medicaments indicated in irritable bowel syndrome (IBD), or with a modulating effect on intestinal motricity, a lipolytic effect, an antiobesity effect, an antidiabetic effect, a psychotropic effect, an antiglaucoma effect, a cicatrizant effect, an antidepressant effect or a tocolytic effect.

10. Compounds of formula (III') 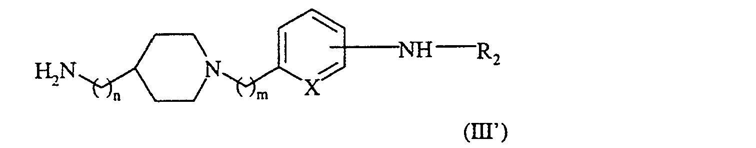 where n, m, X and R₂ are as defined in Claim 1, and their salts or solvates.

11. Compounds of formula (III") 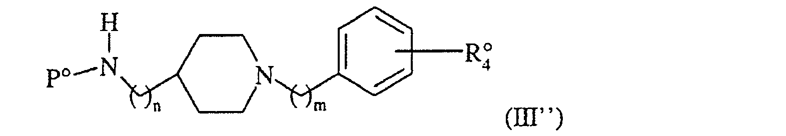 where
P^{o} is a *tert*-butoxycarbonyl group;
n and m are 0, 1 or 2;
R^{o} ₄ is a group chosen from -COO(C₁-C₄)alkyl, -CONR^{o} ₅R^{o} ₆ and -NHSO₂(C₁-C₄) alkyl;
R^{o} ₅ and R^{o} ₆ independently represent a hydrogen atom or a (C₁-C₄) alkyl group;
and their salts or solvates.

12. Compounds of formula (I) according to Claim 1, **characterized in that** they consist of:
• 3-[1-(4-Ethoxycarbonylphenylmethyl)piperidin-4-yl-amino]-1-(4-hydroxyphenoxy)-2-propanol
• 3-[1-(4-Ethoxycarbonylphenyl)piperidin-4-ylamino]-1-(4-hydroxyphenoxy)-2-propanol
• 3-[1-(4-Ethoxycarbonylphenyl)piperidin-4-ylamino]-1-[4-hydroxy-3-(methanesulphonylamino)phenoxy]-2-propanol
• 3-[1-(4-Ethoxycarbonylphenyl)piperidin-4-ylamino]-1-[4-hydroxy-3-(methylsulphinyl)phenoxy]-2-propanol
• 3-[1-(4-N-Butylaminocarbonylphenyl)piperidinylamino]-1-[4-hydroxy-3-(methanesulphonylamino)phenoxy]-(2S)-2-propanol
• 3-[1-(4-N,N-Diethylaminocarbonylphenyl)piperidin-4-ylamino]-1-[4-hydroxy-3-(methanesulphonylamino)phenoxy]-(2S)-2-propanol
• 3-[1-(4-Ethoxycarbonylphenyl)piperidin-4-ylamino]-1-[4-hydroxy-3-(N-butanesulphonylamino)phenoxy]-(2S)-2-propanol
• 3-[1-(4-Ethoxycarbonylphenyl)piperidin-4-ylamino)-1-[4-hydroxy-3-(N-propanesulphonylamino)phenoxy]-(2S)-2-propanol
• 3-[1-(4-Ethoxycarbonylphenyl)piperidin-4-ylamino]-1-[4-hydroxy-3-(methylaminosulphonyl)phenoxy]-(2S)-2-propanol
• 3-[1-(4-Cyanophenyl)piperidin-4-ylamino]-1-[4-hydroxy-3-(N-methanesulphonylamino)phenoxy]-(2S)-2-propanol
• 3-[1-(4-tert-Butoxycarbonylphenyl)piperidin-4-ylamino]-1-[4-hydroxy-3-(N-methanesulphonylamino)phenoxy]-(2S)-2-propanol
• 3-[[1-(4-Ethoxycarbonylphenyl)-4-piperidinyl-methyl]amino]-1-[4-hydroxy-3-(methanesulphonyl-amino)phenoxy]-(2S)-2-propanol
• 5-[((4-Isopropylphenyl)sulphonyl)amino]-2-(4-((3-(4-hydroxyphenoxy)-2-hydroxypropyl)amino)piperidino)pyridine
• 5-[((4-Isopropylphenyl)sulphonyl)amino]-2-(4-((3-(4-benzyloxy-3-(methylsulphinyl)phenoxy)-2-hydroxypropyl)amino)piperidino)pyridine
• 5-[((4-Isopropylphenyl)sulphonyl)amino]-2-(4-((3-(4-hydroxy-3-(methanesulphonylamino)phenoxy)-(2S)-2-hydroxypropyl)amino)piperidino)pyridine
• 5-[((4-Bromophenyl)sulphonyl)amino]-2-(4-((3-(4-hydroxy-3-(methylsulphinyl)phenoxy)-2-hydroxypropyl)amino)piperidino)pyridine
• 3-[1-(4-Ethoxycarbonylphenyl)piperidin-4-ylamino]-1-(4-hydroxy-3-(methylsulphonyl)phenoxy)-(2S)-2-propanol
• 3-[1-[4-((4-Isopropylphenyl)sulphonylamino)phenyl]-piperidinylamino]-1-(4-hydroxy-3-(methanesulphonylamino)phenoxy)-(2S)-2-propanol
or
• 3-[1-[4-((4-Bromophenyl)sulphonylamino)phenyl]-piperidinylamino]-1-(4-hydroxy-3-(methanesulphonylamino)phenoxy)-(2S)-2-propanol and its salts or solvates,

## Patentansprüche

1. Verbindungen der Formel (I) 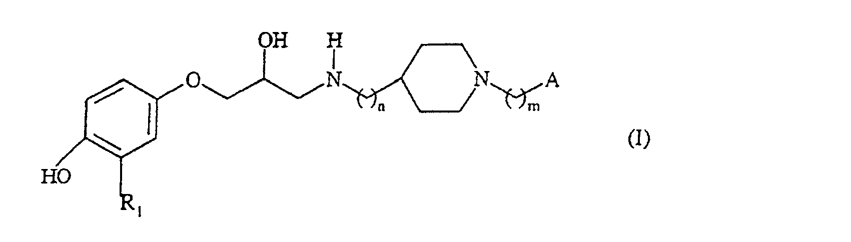 worin
R₁ für ein Wasserstoff- oder Halogenatom oder eine -S(O)_{z}-(C₁-C₄)-Alkyl-,-S (O)_{z}-(C₁-C₄)R₃, -S(O)₂- (C₁-C₄) - Alkyl-, -NHCO(C₁-C₄) -Alkyl-, -CO(C₁-C₄) -Alkyl- oder -NHSO₂-(C₁-C₄) -Alkylgruppe steht;
m und n unabhängig voneinander für 0, 1 oder 2 stehen;
A für eine Gruppe der Formel (a) oder (b) steht: 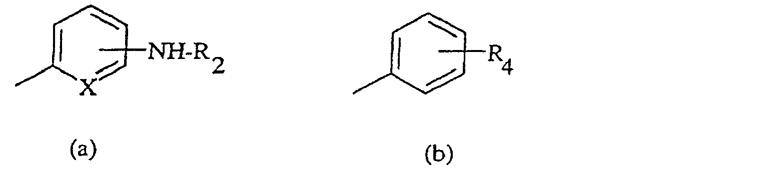
worin:
X für N oder CH steht;
R₂ für eine -SO₂-R₃-, -CO-R₃- oder -CO (C₁-C₄)-Alkylgruppe steht;
R₃ für eine gegebenenfalls durch eine (C₁-C₄) -Alkyl- oder (C₁-C₄)-Alkoxygruppe, ein oder zwei Halogenatome oder einen Heterocyclus substituierte Phenylgruppe steht;
R₄ für ein Wasserstoff- oder Halogenatom oder eine (C₁-C₆)-Alkyl-, (C₁-C₄)-Alkoxy-, -COOH-, -COO(C₁-C₄) -Alkyl-, -CN-, -CONR₅R₆-, -NO₂-, -NHSO₂(C₁-C₄) -Alkyl- oder -SO₂NR₅R₆-Gruppe steht;
z für 1 oder 2 steht;
R₅ und R₆ unabhängig voneinander für ein Wasserstoffatom oder eine (C₁-C₄) -Alkyl-, Phenyl- oder Phenyl(C₁-C₄)-alkylgruppe stehen;
und Salze oder Solvate davon.

2. Verbindungen nach Anspruch 1, worin A für eine Gruppe (a) steht, X für N steht und die Gruppe NHR₂ in der 5-Stellung des Pyridins steht.

3. Verbindungen nach Anspruch 1, worin n und m für Null stehen.

4. Verbindungen nach Anspruch 1, worin es sich bei der (C₁-C₄)-Alkylgruppe um eine Methyl- oder Ethylgruppe handelt.

5. Verbindungen nach Anspruch 1, worin A für eine Gruppe (b) steht und die Gruppe R₄ in der 4-Stellung des Benzols steht.

6. Verbindungen nach Anspruch 1, worin A für eine Gruppe (b) steht und R₄ unter -COOH, -COO (C₁-C₄) - Alkyl, -CN, -NO₂-, -CONR₂R₃-, -NHSO₂ (C₁-C₄) -Alkyl und -SO₂NR₅R₆ ausgewählt ist.

7. Verfahren zur Herstellung von Verbindungen der Formel (I), **dadurch gekennzeichnet, daß** man eine Verbindung der Formel (II) 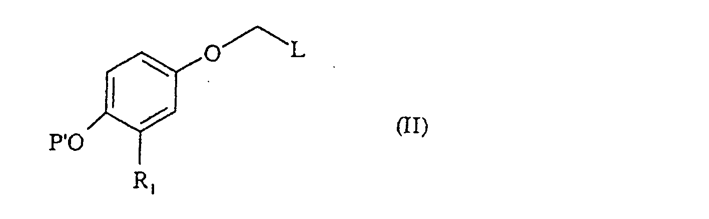 worin R₁ die in Anspruch 1 angegebene Bedeutung besitzt, P' für eine Schutzgruppe steht und L für eine Gruppe der Formel (c) oder (d) steht 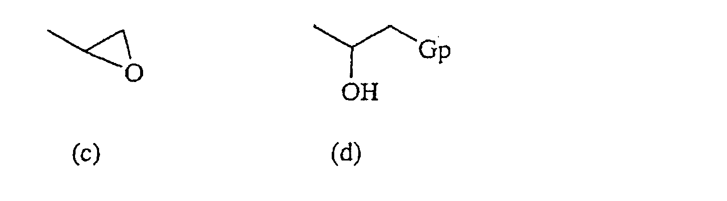 worin Gp für eine Abgangsgruppe steht, mit einem Amin der Formel (III) 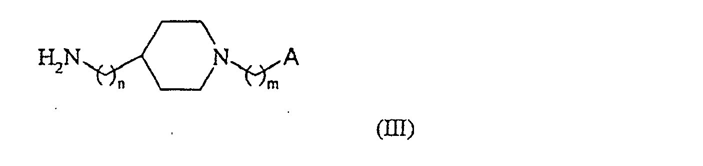 worin n und m die in Anspruch 1 angegebene Bedeutung besitzen, umsetzt, die Gruppe P' abspaltet und gegebenenfalls die so erhaltene Verbindung der Formel (I) in eines ihrer Salze umwandelt.

8. Pharmazeutische Zusammensetzung, enthaltend als Wirkstoff eine Verbindung nach einem der Ansprüche 1 bis 6.

9. Verwendung einer Verbindung nach einem der Ansprüche 1 bis 6 zur Herstellung von Arzneimitteln mit Indikation bei entzündlichen Darmerkrankungen (IBD) oder modulierender Wirkung auf die Darmmotorik, lipolytischer Wirkung, Antiadipositaswirkung, antidiabetischer Wirkung, psychotroper Wirkung, Antiglaukomwirkung, vernarbender Wirkung, antidepressiver Wirkung oder tocolytischer Wirkung.

10. Verbindungen der Formel (III') 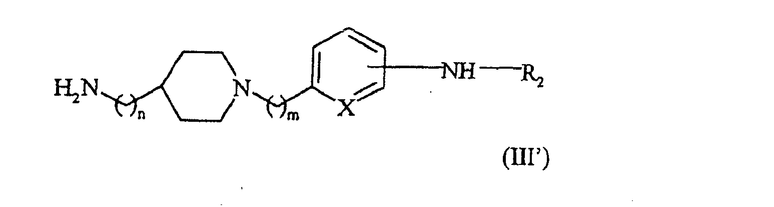 worin n, m, X und R₂ die in Anspruch 1 angegebene Bedeutung besitzen, und Salze oder Solvate davon.

11. Verbindungen der Formel (III'') 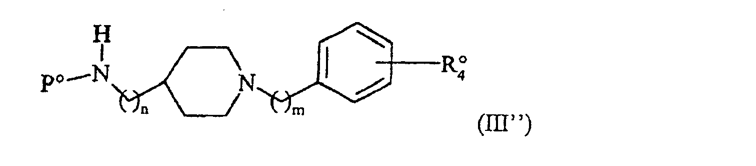 worin
P^{o} für eine tert.-Butoxycarbonylgruppe steht;
n und m für 0, 1 oder 2 stehen;
R₄ für eine unter -COO (C₁-C₄) -Alkyl, -CONR^{o} ₅R^{o} ₆ und -NHSO₂ (C₁-C₄) -Alkyl ausgewählte Gruppe steht;
R^{o} ₅ und R^{o}₆ unabhängig voneinander für ein Wasserstoffatom oder eine (C₁-C₄) -Alkylgruppe stehen;
und Salze oder Solvate davon.

12. Verbindungen der Formel (I) nach Anspruch 1,
**dadurch gekennzeichnet, daß** es sich dabei um:
• 3-[1-(4-Ethoxycarbonylphenylmethyl)piperidin-4-ylamino]-1-(4-hydroxyphenoxy)-2-propanol
• 3-[1-(4-Ethoxycarbonylphenyl)piperidin-4-yl-amino]-1-(4-hydroxyphenoxy)-2-propanol
• 3-[1-(4-Ethoxycarbonylphenyl)piperidin-4-yl-amino]-1-[(4-hydroxy)-3-(methansulfonylamino)-phenoxy]-2-propanol
• 3-[1-(4-Ethoxycarbonylphenyl)piperidin-4-yl-amino]-1-[(4-hydroxy)-3-(methylsulfinyl)-phenoxy]-2-propanol
• 3-[1-(4-N-Butylaminocarbonylphenyl)piperidinyl-amino]-1-[(4-hydroxy)-3-(methansulfonylamino)-phenoxy]-(2S)-2-propanol
• 3-[1-(4-N,N-Diethylaminocarbonylphenyl)-piperidin-4-ylamino]-1-[(4-hydroxy)-3-(methan-sulfonylamino)phenoxy]-(2S)-2-propanol
• 3-[1-(4-Ethoxycarbonylphenyl)piperidin-4-yl-amino]-1-[(4-hydroxy)-3-(N-butansulfonylamino)-phenoxy]-(2S)-2-propanol
• 3-[1-(4-Ethoxycarbonylphenyl)piperidin-4-yl-amino]-1-[(4-hydroxy)-3-(N-propansulfonyl-amino)phenoxy]-(2S)-2-propanol
• 3-[1-(4-Ethoxycarbonylphenyl)piperidin-4-yl-amino]-1-[(4-hydroxy)-3-(methylaminosulfonyl)-phenoxy]-(2S)-2-propanol
• 3-[1-(4-Cyanophenyl)piperidin-4-ylamino]-1-[(4-hydroxy)-3-(N-methansulfonylamino)phenoxy]-(2S)-2-propanol
• 3-[1-(4-tert.-Butoxycarbonylphenyl)piperidin-4-ylamino]-1-[(4-hydroxy)-3-(N-methansulfonyl-amino)phenoxy]-(2S)-2-propanol
• 3-[[1-(4-Ethoxycarbonylphenyl)-4-piperidin-methyl]amino]-1-[(4-hydroxy)-3-(methansulfonyl-amino)phenoxy]-(2S)-2-propanol
• 5-[((4-Isopropylphenyl)sulfonyl)amino]-2-(4-((3-(4-hydroxyphenoxy)-2-hydroxypropyl)amino)-piperidino)pyridin
• 5-[((4-Isopropylphenyl)sulfonyl)amino]-2-(4-((3-(4-benzyloxy)-3-(methylsulfinyl)phenoxy)-2-hydroxypropyl)amino)piperidino)pyridin
• 5-[((4-Isopropylphenyl)sulfonyl)amino]-2-(4-((3-(4-hydroxy-3-methansulfonylamino)phenoxy)-(2S)-2-hydroxypropyl)amino)piperidino)pyridin
• 5-[((4-Bromphenyl)sulfonyl)amino]-2-(4-((3-(4-hydroxy-3-(methylsulfinyl)phenoxy)-2-hydroxypropyl)amino)piperidino)pyridin
• 3-[1-(4-Ethoxycarbonylphenyl)piperidin-4-yl-amino)-1-(4-hydroxy)-3-methylsulfonylphenoxy)-(2S)-2-propanol
• 3-[1-[4-((4-Isopropylphenyl)sulfonylamino)phenyl]-piperidinylamino]-1-(4-hydroxy)-3-methan-sulfonylamino-phenoxy)-(2S)-2-propanol
• 3-[1-[4-((4-Bromphenyl)sulfonylamino)-phenyl]-piperidinylamino]-1-(4-hydroxy)-3-methan-sulfonylamino-phenoxy)-(2S)-2-propanol
und Salze oder Solvate davon handelt.
